# EUROPEAN PATENT SPECIFICATION

(11) **EP 0 226 254 B1**
(45) Date of publication and mention of the grant of the patent: **08.12.1993**
(21) Application number: 86202241.5
(22) Date of filing: 11.12.1986
(51) Int. Cl.: C07H 21/04, C07H 1/08, A61K 31/70, C07G 3/00, C07G 17/00

(54) **Process for obtaining non informational substantially pure polydesoxyribonucleotides having biologic activities, and respective product**
Verfahren zur Herstellung von substantiell reinen nicht für Transpiration geeigneten Polydesoxyribonukleotiden mit biologischen Aktivitäten und respektives Produkt
Procédé d'obtention de polydésoxyribonucléotides substantiellement pures non informationnelles possédant des activités biologiques et produit s'y rapportant

(30) Priority: 12.12.1985 IT 6807485
(43) Date of publication of application: 24.06.1987
(73) Proprietor: FARMIGEA S.P.A., I-56100 Pisa (IT)
(72) Inventor: Bianchini, Pietro, I-41100 Modena (IT)
(74) Representative: Lotti, Giorgio

(56) References cited:
- BE-A- 620 479
- BE-A- 780 872
- CHEMICAL ABSTRACTS, vol. 96, 1982, page 271, no. 48421x, Columbus, Ohio, US; B. SIRRI et al.: "Studies on isolating DNA from human placenta and spleen" & DOGA, SERI C 1980, 4(2), 54-6

## Description

This invention relates to a process for obtaining from the placenta of mammals with emochorial placentation, man inclusive, substantially pure Polydesoxyribonucleotides having no informational activity but provided with biologic activities.

The activities which characterize the product named Polydesoxyribonucleotides and which herein will be called PDRN are the following:
anticomplement activities, activity inhibiting the mediators of the phlogosis, antiinflammatory activity when applied topically, activity inhibiting various aspects of the reactions of immediate (humoral) hypersensivity, fibrinolytic activity when administered in a general way.

Heretofore, the preparation of DNA and PDRN was possible exclusively in laboratory, on a small scale, with high costs.

The process according to this invention is, instead, based on operations partly of general character and partly on a specific character, reproducible and which may advantageously be applied on industrial scale for the production of human or animal DNA and of PDRN, with interesting therapeutic applications.

The nucleic acids and in particular the Desoxyribonucleic acids are the vectors of the genetic information. In fact, the sequence of the four nucleotides, which replace the letters of the genetic code, defines the triplets, each of which identifies an amino acid (Genoma expression).

The Desoxyribonucleic acids exist in the form of a double helice, one of which is not informational in the sense of a capability of "transcription" and is formed by "non sense" triplets, whilst the other one is informational in the sense of a capability of "transcription" and is formed by "sense" triplets.

The state of the art, i.e. the knowledge of the functions of the DNA leads to the conclusion that this polymer has informational functions.

It is therefore surprising that this polymer possess other different pharmacological functions and that it would even be suitable to deprive it of informational abilities (Virus, Oncogenes) in order to render it "safe" and to characterize it better in its "other activities".

The object of the invention is to eliminate these informational capabilities and to consider the Polydesoxyribonucleotide obtained by controlled partial depurination, as being a functionalized polymer capable of binding polypeptides and other substances by eliminating them from the biologic system in which they may cause damages.

The starting material in the process according to this invention is represented by the placenta of emochorial mammals, inclusive man, but it can be applied also to other organs.

The frozen organ is washed with running water in order to defrost it and eliminate the blood which generally is present.

The phases which characterize the process described hereinafter consist of:
1) a thermal inactivation such as to eliminate the action of the degrading endogenous nucleases of DNA which are liberated during the autolytic processes;
2) a proteolysis in controlled conditions;
3) the separation of the residue of the acqueous part;
4) the separation of the PDRN from the liquid by formation complexes or insoluble salts by means of the addition of quaternary bases in solid form (resines) or liquid, or by means of the salification with bivalent metals;
5) the purification of the PDRN after the complexing or elution with solutions of salts of alkaline-earth metals;
6) the controlled partial depurinization of the native PDRN, in order to eliminate the informational capability (virus, oneogenes).

The process according to the present invention will now be described in detail.

A saline solution whose composition is described in the examples which follow hereinafter, is prepared separately in a vessel or a reactor of suitable capacity.

Also separately there is prepared a suspension of proteolytic enzymes.

After the defrosting, the organ is minced and the obtained pulp is introduced into the vessel or reactor containing the saline solution, the whole being maintained incontinuos stirring.

The pH is corrected to a value between 6 and 7 and, still under stirring, the suspension of proteolytic enzymes is added thus raising the temperature to 50^{o}C.

The mass is maintained under such conditions till the proteolysis is completed, which is obtained within 16-20 hours, depending on the enzimes used.

The pH is modified to between 5,5 and 5,8 and the whole is brought to ebullition for few minutes.

The mixture is hot filtered rapidly and the limpid or slightly opalescent liquid is brought to neutral pH.

This liquid is the FIRST INTERMEDIATE.

From the FIRST INTERMEDIATE the raw POLYDESOXYRIBONUCLEOTIDES can be obtained by different systems.
1) precipitation of PDRN by means of polar solvents;
2) precipitation of PDRN by means of Quaternary Ammonium Bases;
3) precipitation of the PDRN by means of alkaline-earth metal salts;
4) precipitation of the PDRN by means of heavy metal salts;
5) retention of the PDRN on ion exchange resins;
6) separation of the PDRN by means of lattice resins.
These systems may be used separately or in sequence and are described in detail hereunder.
1) Precipitation of the PDRN by means of polar solvents
   The addition of polar solvents, such as for exemple acetone, methanol, ethanol, to the FIRST INTERMEDIATE gives rise to the precipitation of a tacky mass which represents the SECOND INTERMEDIATE.
2) precipitation of the PDRN from the FIRST INTERMEDIATE by means of Quaternary Ammonium bases; hereinafter referred to as BQA, with a suitable saline concentration, followed by a decomplexing of the adduct and recovery of the bases with precipitation of the PDRN by means of polar solvents, as described in paragraph 1).
3) Precipitation of the PDRN from the FIRST INTERMEDIATE by means alkaline-earth metal salts, such as Ca or Mg, in suitable dilution and at a low temperature, and in the presence of polar solvents.
   The precipitate which has been formed after a suitable waiting time, collected and treated with high molarity solutions of Sodium chloride in the presence of sequestrating agents of the EDTA type, is precipitated again by means of polar solvents as in paragraph 1).
4) Precipitation of the PDRN from the FIRST INTERMEDIATE by means of heavy metal salts, such as for example salts of Zn, at ambient temperature or hot. The precipitate thus formed is treated by means of a passage of the suspension onto a column of ion exchange resin. The percolate is made to precipitate by means of polar solvents, as described in paragraph 1).
5) Retention of the PDRN on ion exchange resins.
   By percolating the suitably diluted FIRST INTERMEDIATE onto an appropriate aionic resin, thid latter retains the PDRN and exludes (or with the exclusion of) the other substances.After the washing of the resin, the PDRN is eluted with a saline solution at suitable pH and molarity. The eluate is made to precipitate by means of polar solvents, as described in paragraph 1).
6) Separation of the PDRN by means of lattice resins.
   The PDRN may be recovered from the FIRST INTERMEDIATE by by molecular filtration using a lattice resin of various types but such as to allow; for example, the exclusion of small molecules. The separation can be carried out on a column or in bulk. The excluded liquid contains the PDRN which is made to precipitate by means of polar solvents, as described in paragraph 1).

The precipitate obtained by the six methods indicated hereinabove, and which is called SECOND INTERMEDIATE or RAW PDRN contains various quantities, depending on the methods used, of other polymers of high molecular weight, such as, for exemple, polysaccharides (hialuronic acid, dermatasolfates, condroitinosolfates, heparin) from which the PDRN may be separated by purification, the process of which is described hereunder.

This process is subdivided into the following phases:
a) Purification of the PDRN
   The purification of the PDRN is necessary, independently from the process used to obtain the SECOND INTERMEDIATE. More particularly, the process of purification is carried out as follows: the material of the SECOND INTERMEDIATE is dissolved or solususpended in water, the aqueous solususpension obtained with the SECOND INTERMEDIATE is treated, in the presence of phosphate buffer pH = 7,6, with a molar solution of a salt of magnesium and a suitable quantity of a low temperature polar solvent.
   The precipitate formed, collected and suspended in a sodic EDTA solution, after a dialysis, is made to precipitate again by means of polar solvents.
b) Controlled depurinization with partial elimination of the purinic bases Adenine and Guanine.
   The acqueous solususpension of the SECOND INTERMEDIATE is treated, with a hot treatment and for an appropriate period of time, acetate buffer pH = 4,2. At the termination of the treatment, after the correction of the pH to values proximate to the neutrality, the PDRN is made to precipitate by means of polar solvents.
c) Fractionation of the biologically active molecular species is performed by specific absorption of the product obtained by partial depurinization, on columns of hydroxylapatite in phosphate buffer at increasing molarities. At phosphate buffer molarity of 0,02 M, pH = 7,2. Inactive fragments are eluted. At the molarity of 0,12 M the single helice PDRN comes out, whilst at a molarity of 0,45 M also the double-helice PDRN; more intensively absorbed, comes out. Hence, only the PDRN which is eluted at between 0,1 and 0,45 Molar of phosphate buffer is active.
   The material, after an extensive dialysis and concentration, is made to precipitate by means polar solvents (ethano-methanol-acetone) or lyophilized.
EXAMPLE
- Preparation of the FIRST INTERMEDIATE
   100 kilograms of Human Placenta have been defrosted, washed with running water, deprived of umbilical cords and membranes and turned into a pulp (by means of a mincer or equivalents).
   Thereafter they have been homogenized with a solution (40-50 litres) 2,5 M Sodium chloride and 0,07 M of thiosulphate. After a correction of the pH 6, a soluble Papain Merck was added in the proprtion of 1% relative to the substratum and the mass was left to proteolize at 50^{o}C. After accomplishment of the proteolysis and correction of the pH to 5-5,5, the mass was brough to ebollition and hot filtered. The liquid obtained (A)has been brought to pH 7 and it constitutes the FIRST INTERMEDIATE.
- Preparation of the SECOND INTERMEDIATE
   1) Precipitation of the PDRN by means of polar solvents. The liquid (A) - FIRST INTERMEDIATE - has been made to precipitate by means of 2 volumes of Ethanol 95 - 97 or Methanol, and centrifuged.
      The obtained solid, washed repeatedly with Ethanol or with Methanol, has been dried under at 40° C vacuum. It forms the SECOND INTERMEDIATE.
      The content of PDRN of such SECOND INTERMEDIATE (obtained by means of ethanol or methanol ) does not exceed 30%.
   2) Precipitation of the PDRN by means if Quaternary Ammonium Bases.
      The liquid (A) - FIRST INTERMEDIATE - diluted till a saline concentration of 0,2 Molar is obtained, has been made to precipitate by means of an excess of a salt of Quaternary Ammonium (BQA) at 50^{o}C and left to rest for some hours. The amount (Quaternary Ammonium salt)had been predetermined on a sample and varies depending on the Ammonium salt used.
      The solid obtained after centrifugation has been solususpended in a 3M solution of sodium chloride, in a quantity sufficient to discomplex it, (a volume of approximately 10 times the weight of the complex has been used V/W=10),and made to precipitate by means of two volumes of Ethanol.
      The solid obtained after centrifugation and repeated washings with Ethanol, has been dried under vacuum at a temperature of 40^{o}C.
      The SECOND INTERMEDIATE thus obtained contains 60-70% of PDRN.
   3) Precipitation of the PDRN by means of salts of alkaline-earth metals.
      The liquid (A) has been cooled at room temperature, brought to a pH = 7,8-8, added with a quantity of phosphate buffer pH = 7,8 so as to obtain a final concentration of 0,005M/litre, and with a Magnesium chloride till a concentration of 0,005 Molar is obtained, and then it has been made to precipitate by means of 0,7 volumes of ethanol, cooled at -20 for at least half an hour and centrifuged. The solid obtained has been suspended in a 0,1M solution of sodic EDTA and dialized first un running watern then in distilled water for at least 24 hours. After a dialysis, the gelatinous suspension has been made to precipitate by means of 2 volumes of ethanol and washed with the same solvent and then dried at 40^{o}C under vacuum in a stove. Such SECOND INTERMEDIATE has a content of PDRN of 70%.
- Purification of the PDRN
   The SECOND INTERMEDIATE has been suspended in distilled water at a concentration of 2%, and after addition of an amount of phosphate buffer pH 7,8 till a 0,005 Molar concentration is obtained, the PDRN has been made to precipitate as a salt of Mg by adding Magnesium chloride till obtaining a concentration of 0,006 Molar and 0,7 volumes of ethanol. The mixture, after cooling at -20^{o}C for at least half an hour, has been centrifuged.
   The solid has been solususpended in a 0,1 Molar solution of sodic EDTA and dialized in tubes KALLE dialysis (Kal : 30 - Lange : 25 : Menge :1) or by means of an apparatus for inverted osmosis first with running water, then with distilled water (25-30 times the volume of the liquid to be dialized) for at least 24 hours.
   The dialized solution has been made to precipitate by means of 2 volumes of Ethanol,then centrifuged, washed repeatedly with Ethanol and dried under vacuum at 40^{o}C.
   The product obtained contains 85-90% of PDRN and a humidity of 9-10%. This operation has to be repeated at least two times if the intermediate obtained according to paragraph 1) is used.
- Depurination of the PDRN.
   The purified PDRN powder - at the concentration of 1% - has been suspended in acetate buffer 0,1M pH = 4,2 and heated at 70^{o}C for 2 hours.
   After cooling with running water, the pH has been corrected to 7,2 and the solususpension has been made to precipitate by means of 2 volumes of Ethanol after having previously been salted with sodium chloride.
   After centrifugation, the solid has been washed 2-3 times with Ethanol and dried under vacuum at 40^{o}C.
   Loss of weight: 5-10%.
   Loss of anticomplement activity: 10-12% besides the loss of weight.
   The controlled partial depurination gives rise after 1 hour to the loss of 0,8% of G and 0,6% of A, so that there arises a total loss of 1,4 bases on 100, i.e. one purine base every 75 total bases.
   The segment of DNA between two missing bases is thus of about 4,95 . 10⁴ Daltons on the average.
   The smallest segment of DNA capable of coding for an oncogene product is of 2,31 . 10⁵ Daltons, i.e. 350 bases.
   The controlled partial depurination forming the subject matter of the present invention ensures for the product the loss of the informational capability which practically gives rise to the destruction of oncogenes and viral genes, without however loosing the capability of the PDRN to maintain its complement inhibiting activity, to inhibit some chimical mediators of the phlogosis, to inhibit some reactions of immediate hypersensitivity, to inhibit the migration of leucocytes and the induction of granuloma due to a foreign body, and, in human patology, to possess therapeutic efficiency in the cervico-vaginites due to radiant treatment, or to senile dystrophia, in the series of diathermocoagulation, in the ectropion and transformation's areas.

## Claims

1. A process for the obtainment of non informational, substantially pure Polydesoxyribonucleotides (PDRN) having biologic activities, characterized in being composed of the following phases:
a) reducing to a pulp the starting material formed by the placenta of emochorial mammals;
b) thermal inactivation such as to eliminate the action of degrading endogenous nucleases of DNA which are liberated during the autolitic processes;
c) proteolysis of the material in controlled conditions;
d) separation, from the digest, of the residue, which is not soluble,by the aqueous part;
e) separation of the PDRN from the liquid throug formation of complexes by means of the addition of quaternary bases in solid or liquid form, or by means of the salification with bivalent metals:
f) purification of the PDRN after decomplexing or elution with solutions of alkaline-earth metal salts;
g) controlled partial depurinization of the native PDRN, in order to eliminate the informational capability (virus-oncogenes).

2. A process as claimed in Claim 1, characterized in that the purification of PDRN in the phase f)comprises first its suspension in distilled water and, after addition of a quantity of phosphate buffer, its treatment with a molar solution of a salt of magnesium by means of a low temperature and a polar solvent.

3. A process as claimed in Claim 1, characterized in that the controlled partial depurination of the PDRN during the phase g) comprises first its hot treatment with acetate buffer and, after the correction of the pH to values proximate to the neutrality, its precipitation by means of polar solvents.

4. Non informational, substantially pure Polydesoxyribonucleotide obtainable in accordance with the process described in the preceding Claims, characterized in possessing:
- Anticomplement activity in vitro
- Activity inhibiting the chemical mediators of the phlogosis in vitro
- Activity inhibiting the phlogosis due to carragenine and to foreign bodies
- Activity inhibiting the migration of the leucocytes in vivo
- Therapeutic activity on the cervico-vaginites, lesions due to diathermocoagulation, ectropion and transformation's areas.

5. Use of non informational, substantially pure polydesoxyribonucleotides obtainable in accordance with the process described in Claims from 1 to 3 for obtaining a preparation with anticomplement activities.

6. Use of non informational, substantially pure polydesoxyribonucleotides obtainable in accordance with the process described in Claims from 1 to 3 in order to obtain a preparation with activities inhibiting the chemical mediators of the phlogosis.

7. Use of non informational substantiallty pure polydesoxyribonucleotides obtainable in accordance with the process described in claims from 1 to 3 in order to obtain a preparation with an activity inhibiting the phlogosis due to carragenine and to foreign bodies.

8. Use of non informational substantially pure polydesoxyribonucleotides obtainable in accordance with the process described in Claims from 1 to 3 in order to obtain a preparation with an activity inhibiting the migration of the leucocytes.

9. Use of non informational substantially pure polydesoxyribonucleotides obtainable in accordance with the process described in Claims from 1 to 3 in order to obtain a preparation with an activity inhibiting the reactions of immediate hypersensitivity.

10. Use of non informational substantially pure polydesoxyribonucleotides obtainable in accordance with the process described in Claims from 1 to 3 in order to obtain a preparation with a therapeutic activity on the cervico-vaginites, lesions caused by diathermocoagulation, ectropion and transformation's areas.

## Patentansprüche

1. Verfahren zur Herstellung von im wesentlichen reinen, nicht für die Information geeigneten Polydesoxyribonukleotiden (PDRN) mit biologischen Aktivitäten,
**gekennzeichnet durch**
die folgenden Phasen:
a) Reduzieren des durch die Plazenta emochorialer Säugetiere gebildeten Ausgangsmaterials zu einem Brei,
b) thermische Inaktivierung, um die abbauende Wirkung endogener DNA-Nukleasen, die während des autolytischen Verfahrens freigesetzt werden, zu eliminieren,
c) Proteolyse des Materials unter kontrollierten Bedingungen,
d) Abtrennen des nicht-löslichen Restes aus dem Verdauten in der wäßrigen Phase,
e) Abtrennen der PDRN aus der Flüssigkeit durch Komplexbildung mittels der Zugabe quaternärer Basen in fester oder flüssiger Form oder durch die Salzbildung mit bivalenten Metallen,
f) Reinigen der PDRN nach Dekomplexieren oder Eluieren mit Lösungen von Erdalkalimetallsalzen,
g) kontrollierte Teildepurinisierung der nativen PDRN, um die Informationsfähigkeit zu eliminieren (Virus-Onkogene).

2. Verfahren nach Anspruch 1,
**dadurch gekennzeichnet,** daß
das Reinigen der PDRN in Phase f) zuerst deren Suspendierung in destilliertem Wasser und, nach Zugabe einer Menge an Phosphatpuffern, deren Behandlung mit einer molaren Lösung eines Magnesiumsalzes bei niederen Temperaturen und einem polaren Lösungsmittel umfaßt.

3. Verfahren nach Anspruch 1,
**dadurch gekennzeichnet,** daß
die kontrollierte Teildepurinisierung der PDRN während der Phase g) zuerst deren Wärmebehandlung mit Acetatpuffer und, nach Einstellen des pH-Wertes auf einen Wert nahe dem Neutralpunkt, deren Präzipitation durch polare Lösungsmittel umfaßt.

4. Im wesentlichen reine, nicht für die Information geeignete Polydesoxyribonukleotide, erhältlich gemäß dem Verfahren nach einem der vorhergehenden Ansprüche,
**gekennzeichnet durch**
Besitz an
- in vitro-Antikomplementaktivität,
- in vitro-Aktivitätsinhibierung der chemischen Mediatoren der Phlogosis,
- Aktivitätsinhibierung der Phlogosis, die von Carragenin und Fremdkörpern herrührt,
- in vivo-Aktivitätsinhibierung der Leukozytenmigration,
- therapeutische Aktivität gegenüber der Cervigo-Vaginitis, durch Diathermokoagulation, Ektropion und Transformationsbereiche hervorgerufene Läsionen.

5. Verwendung von im wesentlichen reinen, nicht für die Information geeigneten Polydesoxyribonukleotiden, erhältlich gemäß dem Verfahren nach den Ansprüchen 1 bis 3, unter Erhalt einer Präparation mit Antikomplementaktivität.

6. Verwendung von im wesentlichen reinen, nicht für die Information geeigneten Polydesoxyribonukleotiden, erhältlich gemäß dem Verfahren nach den Ansprüchen 1 bis 3,unter Erhalt einer Präparation mit Aktivitätsinhibierung der chemischen Mediatoren der Phlogosis.

7. Verwendung von im wesentlichen reinen, nicht für die Information geeigneten Polydesoxyribonukleotiden, erhältlich gemäß dem Verfahren nach den Ansprüchen 1 bis 3, unter Erhalt einer Präparation mit Aktivitätsinhibierung der Phlogosis, die von Carragenin und Fremdkörpern herrührt.

8. Verwendung von im wesentlichen reinen, nicht für die Information geeigneten Polydesoxyribonukleotiden, erhältlich gemäß dem Verfahren nach den Ansprüchen 1 bis 3, unter Erhalt einer Präparation mit Aktivitätsinhibierung der Leukozytenmigration.

9. Verwendung von im wesentlichen reinen, nicht für die Information geeigneten Polydesoxyribonukleotiden, erhältlich gemäß dem Verfahren nach den Ansprüchen 1 bis 3, unter Erhalt einer Präparation mit Aktivitätsinhibierung der Reaktionen von Sofort-Hypersensitivität.

10. Verwendung von im wesentlichen reinen, nicht für die Information geeigneten Polydesoxyribonukleotiden, erhältlich gemäß dem Verfahren nach den Ansprüchen 1 bis 3, unter Erhalt einer Präparation mit einer therapeutischen Aktivität gegenüber der Cervigo-Vaginitis, durch Diathermokoagulation, Ektropion und Transformationsbereiche hervorgerufene Läsionen.

## Revendications

1. Procédé d'obtention de Polydésoxyribonucléotides (PDRN) sensiblement purs, non informationnels, et à activités biologiques, caractérisé en ce qu'il comporte les phases suivantes:
a) une réduction, en pulpe, de la matière de départ formée par le placenta de mammifères de type émochorial ;
b) une inactivation thermique de manière à éliminer l'effet des nucléases endogènes dégradantes de DNA qui sont libérées pendant les processus autolytiques;
c) une protéolyse de la matière dans des conditions réglées;
d) une séparation, à partir du digesté, du résidu, qui n'est pas soluble, par la partie aqueuse;
e) une séparation des PDRN du liquide par formation de complexes au moyen d'une addition de bases quaternaires sous forme solide ou liquide, ou au moyen d'une salification par des métaux bivalents;
f) une purification des PDRN après décomplexion ou élution avec des solutions de sels de métaux alcalino-terreux;
g) une dépurination partielle réglée des PDRN natifs, afin d'éliminer la capacité informationnelle (virus-oncogènes).

2. Procédé selon la revendication 1, caractérisé en ce que la purification des PDRN lors de la phase f) comprend en premier lieu leur suspension dans de l'eau distillée, et après l'addition d'une quantité d'un tampon de phosphate, leur traitement à l'aide d'une solution molaire d'un sel de magnésium au moyen d'une basse température et d'un solvant polaire.

3. Procédé selon la revendication 1, caractérisé en ce que la dépurination partielle réglée des PDRN pendant la phase g), comprend en premier lieu leur traitement à chaud, avec un tampon d'acétate, et, après la correction du pH à des valeurs proches de la neutralité, leur précipitation au moyen de solvants polaires.

4. Polydésoxyribonucléotide non informationnel, sensiblement pur, et susceptible d'être obtenu selon le procédé des revendications précédentes, caractérisé en ce qu'il possède :
- une activité anticomplément in vitro
- une activité inhibant les médiateurs chimiques de la phlogose in vitro
- une activité inhibant la phlogose due à la carragénine et à des corps étrangers
- une activité inhibant la migration des leucocytes in vivo
- une activité thérapeutique des cervico-vaginites, des lésions dues à une diathermocoagulation, de l'ectropion et des zones de transformation.

5. Utilisation de polydésoxyribonucléotides non informationnels, sensiblement purs, et susceptibles d'être obtenus selon le procédé défini aux revendications 1 à 3, afin d'obtenir une préparation à activités anticompléments.

6. Utilisation de polydésoxyribonucléotides non informationnels, sensiblement purs, et susceptibles d'être obtenus selon le procédé défini aux revendications 1 à 3, afin d'obtenir une préparation à activité inhibant les médiateurs chimiques de la phlogose.

7. Utilisation de polydésoxyribonucléotides non informationnels, sensiblement purs, et susceptibles d'être obtenus selon le procédé défini aux revendications 1 à 3, afin d'obtenir une préparation à activité inhibant la phlogose due à la carragénine et à des corps étrangers.

8. Utilisation de polydésoxyribonucléotides non informationnels, sensiblement purs, et susceptibles d'être obtenus selon le procédé défini aux revendications 1 à 3, afin d'obtenir une préparation à activité inhibant la migration des leucocytes.

9. Utilisation de polydésoxyribonucléotides non informationnels, sensiblement purs, et susceptibles d'être obtenus selon le procédé défini aux revendications 1 à 3, afin d'obtenir une préparation à activité inhibant les réactions d'hypersensibilité immédiate.

10. Utilisation de polydésoxyribonucléotides non informationnels, sensiblement purs, et susceptibles d'être obtenus selon le procédé défini aux revendications 1 à 3, afin d'obtenir une préparation à activité thérapeutique sur les cervico-vaginites, les lésions causées par la diathermocoagulation, l'ectropion et les zones de transformation.
